# EUROPEAN PATENT APPLICATION

(11) **EP 2 543 339 A1**
(43) Date of publication of application: **09.01.2013**
(21) Application number: 11172704.6
(22) Date of filing: 05.07.2011
(51) Int. Cl.: A61F 2/00

(54) **Surgical implant, in particular for use as a hernia repair implant**

(71) Applicant: Aesculap AG, 78532 Tuttlingen/Donau (DE); B. Braun Surgical, S.A., 08191 Rubi (Barcelona) (ES)
(72) Inventor: Odermatt, Erich, 8200 Schaffhausen (CH); Pfeiffer, Ruth Verena, 08173 Sant Cugat del Vallès (Barcelona) (ES)
(74) Representative: Patentanwälte Ruff, Wilhelm, Beier, Dauster & Partner

(57) **Abstract**

The present invention relates to a surgical implant (100), in particular for use as a hernia repair implant, comprising a base component (110) and a plug component (120) wherein the base component (110) is adapted to be located beneath a hernia orifice and the plug component (120) is adapted to be placed within a hernia orifice.

Further, the invention relates to a surgical combination, in particular a surgical kit, comprising a surgical implant (100) according to the invention and a delivery instrument.

## Description

The present invention relates to a surgical implant which is especially useful for hernia repair management and to a surgical combination comprising the implant.

Hernia surgery commonly provides for tension repair techniques and tension free repair techniques in order to treat hernia defects. A plug for surgery and treatment of hernioplasty having a base and a flattened blunted apex with grounded shape is know from EP 0 888 756 A2.

Tension repair techniques are dependent on the usage of sutures or wires. These techniques typically create extreme tension on the surrounding tissue in order to close the hernia defects. This may result in swelling, pain and a limited mobility of the patients concerned. Moreover, the tension applied on the surrounding tissue may impair a complete and effective healing of the wound edges. This in turn may lead to a higher failure rate with recurrent hernia being often larger and more complex.

Presently, there are basically three techniques known which may be contemplated as tension free techniques.

The laparoscopic hernia repair techniques such as transabdominal preperitoneal patch technique and intraperitoneal onlay mesh technique involve deploying a mesh onto a hernia defect from inside the abdomen. This requires deployment instruments being stabbed through the abdominal wall, thereby risking injury to the intestine. These instruments allow for positioning the mesh under the hernia defect, held in place with sutures, staples or glues. Though, the post-operative discomfort is relatively small, the recurrence rate is not irrelevant.

The Lichtenstein repair technique typically requires a mesh which is applied over the hernia defect and sewn in place. In contrast to the laparoscopic hernia repair technique, this surgery may be done under local anesthesia, and direct visualisation of the hernia, without using a deployment instrument. However, also the Lichtenstein technique may not prevent the formation of recurrent hernias to a certain extent.

The plug hernia repair technique typically provides for placing a mesh through the defect. Thereafter, the mesh fills the hernial orifice. Afterwards, an additional sheet of mesh is included over the defect as an insurance reinforcement. The latter mesh is placed in a completely tension free fashion. On the one hand, there is no tension and commonly only a minimal amount of surgical dissection is necessary resulting in little post-operative pain. On the other hand, the mesh placed within the hernia defect may be prone to shrinkage and/or movements, thereby favouring the recurrence of hernia.

Therefore, it is an object of the present invention to provide a surgical implant which may be useful for treating hernia defects, thereby avoiding withdrawals of hernia implants known from the prior art. In particular, the surgical implant shall be applicable within a tension free hernia repair technique.

This object is achieved by a surgical implant according to independent claim 1. Embodiments of the implant are reflected in dependent claims 2 to 18. Besides, the invention pertains to a surgical combination having the features of independent claim 19. The subject matter of all claims is hereby made to the content of the present description by explicit reference.

The surgical implant according to the present invention is in particular useful for hernia repair management. The implant comprises a base component and a plug component. Preferably, the base component is adapted to be located beneath a hernia orifice, while the plug component is preferably adapted to be placed within a hernia orifice.

A hernia is typically understood as the protrusion of an organ or a fascia thereof through the wall of a cavity which normally contains the complete organ including the fascia thereof. Typically, hernias are originated from protrusions of organs or fascias thereof through the abdominal wall.

The phrase "the base component is adapted to be located beneath a hernia orifice" as used according to the present invention means that the base component is adapted to be located towards the inner surface of a cavity wall which normally, i.e. in a healthy subject, prevents protrusion of an organ or fascia thereof. The phrase "inner surface of a cavity wall" as used according to the invention means the surface which is directed to the interior of the cavity.

The phrase "hernia orifice" as used according to the present invention means an orifice in the wall of a cavity as mentioned in the preceding paragraphs which is either due to protrusion of an organ (or a fascia thereof) or due to an incision made by a surgeon in order to facilitate or improve treatment of hernia.

In a preferred embodiment, the surgical implant comprises an integral structure which is composed of the base component, plug component, and, if need be, further components such as a gripping element, fixating elements, positioning elements such as a pocket or pouch, and/or additives as will be described in the following in more detail.

Typically, the base component and the plug component are attached or assembled to each other, thereby yielding an integral implant structure. More specifically, the base component and the plug component may be attached to each other by means of bonding, gluing, welding, particularly ultrasound welding, heating, thermal fixing, clipping, stapling or combinations thereof. In addition or alternation, the base component and the plug component may be attached to each other by textile construction such as stitching, knitting, interlooping, intermeshing; or the like. Further, the base component and the plug component may be attached to each other only partly or over whole surfaces thereof.

The base component preferably acts as a reinforcing or load-bearing component, in particular as a carrier or support component. For example, the base component may act as a carrier component for additives such as cells and/or active agents such as platelet-rich plasma. The base component preferably exhibits sufficient mechanical characteristics such as strength, in particular tensile strength, breaking load, stiffness, or the like. Advantageously, the base component contributes to a secure attachment of the implant within a patient's body. As mentioned before, it is especially preferred for the base component to be located or placed beneath a hernia orifice.

The location of the base component beneath a hernia orifice may be supported by means of suitable fixating techniques such as tissue adhesives, sutures, clips and/or retaining elements. With respect to retaining elements, it is referred to the following description.

In a preferred embodiment, the base component is present as a two-dimensionally shaped body.

According to a further embodiment, the base component has a textile structure. More specifically, the base component may be a textile fabric, in particular a flat or two-dimensional textile fabric. For example, the base component may be selected from the group consisting of a mesh or open meshed fabric, a knitted fabric, in particular a warp knitted fabric, a hosiery, a non-woven fabric, a felt, a braided fabric and combinations thereof.

Preferably, the base component is a textile mesh, netting or open meshed fabric, preferably in the form of a knitted fabric, particularly in the form of a warp knitted fabric. The terms "mesh" and "open meshed fabric" may be used interchangeably or synonymously in the following description and are both synonyms for the term "netting".

Alternatively, the base component may have a non-textile structure. For example, it may be within the scope of the present invention that the base component is present in the form of a membrane, sponge, foam, mat, layer, coating, film, foil, and combinations thereof.

Further, the base component may be lyophilized or a lyophilized formed or shaped body. With respect to useful shaped bodies, reference is in particular made to the preceding embodiment.

In principle, the base component may be non-absorbable, partly absorbable or absorbable. In other words, the base component may comprise or may be made of a non-absorbable, partly absorbable or absorbable material.

Preferably, the base component is non-absorbable. This is advantageous inasmuch as the base component may contribute to a stabilisation of the closed hernia defect zone over the whole implantation time.

The material is preferably a polymer or a mixture of polymers. More specifically, the material may be a chemically synthesized, recombinantly synthesized and/or naturally occurring polymer or a mixture of such polymers. Moreover, the material may be a copolymer.

A copolymer as used according to the present invention relates to a polymer which is composed of at least two different monomer units. Thus, polymers having, by way of example, three or four different monomer units, i.e. terpolymers or tetrapolymers, may also be encompassed by the term "copolymer" as used according to the present invention. For example, the material may be a terpolymer, in particular a triblock terpolymer.

In a more specific embodiment, the base component comprises or is made of a material which is selected from the group consisting of polyolefins, polyesters, polyurethanes, in particular thermoplastic polyurethanes, polyamides, polyhydroxyalkanoates, biopolymers, i.e. naturally occurring polymers, copolymers, in particular terpolymers, thereof, salts thereof, stereoisomers thereof, and combinations thereof.

Useful biopolymers may be selected from the group consisting of proteins, extracellular proteins, fibrous proteins, polysaccharides, alkyl celluloses, hydroxylalkyl celluloses, carboxyalkyl celluloses, mucopolysaccharides, salts thereof, stereoisomers thereof, and combinations thereof.

In a more specific embodiment, the base component comprises or is made of a material which is selected from the group consisting of polyethylene, low-density polyethylene, high-density polyethylene, high-molecular-weight polyethylene, ultra-high-molecular-weight polyethylene, polypropylene, polyethylene terephthalate, polypropylene terephthalate, polybutylene terephthalate, polyamide 6, polyamide 6-6, polyamide 6-12, polyamide 12, polytetrafluorethylene, polyvinylidene dichloride, polyvinylidene difluoride, polytetrafluorpropylene, polyhexafluorpropylene, polyvinyl alcohol, polyglycolide, polylactide, polydioxanone, polyhydroxybutyrate, poly-3-hydroxybutyrate, poly-4-hydroxybutyrate, polytrimethylenecarbonate, poly-ε-caprolactone, collagen, gelatine, elastin, reticulin, fibronectin, laminin, fibrin, fibrinogen, albumin, starch, amylose, amylopectin, dextran, cellulose, methylcellulose, carboxymethylcellulose, chitosan, hyaluronic acid, dextran sulfate, heparin, heparan sulfate, chondroitin sulfate, dermatan sulfate, copolymers, particularly terpolymers, thereof, stereoisomers thereof, salts thereof, and combinations thereof.

A useful homopolymer for the base component is poly-4-hydroxybutyrate, in particular due to its capability of long-term absorption.

A useful copolymer for the base component is a copolymer made of glycolide and lactide, in particular having a weight ratio from 9 : 1 to 1 : 9. When collagen is used for the base component, the collagen may be selected from the group consisting of collagen type I, type II, type III, type IV, type V, type VI, type XI, salts thereof, modifications thereof, and combinations thereof. The collagen may be present in a native and/or denatured condition. Typically, the collagen is of xenogenic, in particular porcine, bovine and/or equine, preferably bovine, origin.

Additionally or alternately, collagen may also be of recombinant origin.

Preferably, the base component is a mesh or an open meshed fabric, in particular a non-absorbable mesh or open meshed fabric, preferably made of a polyolefin, more preferably made of polypropylene.

Further, the base component may be a light-weight open meshed fabric, in particular having an area weight between 15 and 70 g/m², in particular 20 and 60 g/m², preferably 25 and 50 g/m².

More specifically, the base component may be a textile mesh which is commercially available under the name Optilene^{®} Mesh LP and/or Optilene^{®} Mesh Elastic. Optilene^{®} Mesh LP is an open meshed fabric made of polypropylene monofilaments, having an area weight of 36 ± 5 g/m², a mesh size of about 1 mm and a pore density of about 112 pores/cm². Optilene^{®} Mesh Elastic is an open meshed fabric made of polypropylene monofilaments having an area weight of 48 ± 7 g/m², a mesh size of 3,6 x 2,8 mm and a pore density of about 60 pores/cm².

In an alternate embodiment, the base component is a non-woven fabric, a felt or a membrane, preferably a membrane, including or predominantly including a protein such as collagen, gelatine, albumin and/or salts thereof. The collagen is preferably selected from the group consisting of collagen type I, collagen type III, salts thereof, stereoisomers thereof, modifications thereof, and combinations thereof.

Further, the base component may be or include a biological membrane, particularly a membrane of xenogenic origin, for example of porcine, bovine and/or equine origin. More specifically, the base component may be or include a pericardium membrane or a synthetically produced collagen or gelatine membrane.

In a further embodiment, the base component, in particular being formed as a synthetic membrane or synthetic film such as a collagen membrane or collagen film, may be cross-linked or non-cross-linked.

In a further embodiment, the base component is a non-woven fabric made of polytetrafluorethylene (PTFE).

In a further embodiment, the base component may be a composite, i.e. a structure which is composed of at least two different structures and/or of at least two different materials.

More specifically, the composite may comprise a textile structure, preferably formed as mesh or open meshed fabric, and a non-textile structure such as a non-woven fabric, felt or membrane, preferably a membrane.

In a further embodiment, the base component is a composite comprising a non-absorbable structure, in particular a non-absorbable textile structure, and an absorbable structure, in particular an absorbable non-textile structure.

With respect to useful structures and/or materials for the composite, reference is made in its entirety to the structures and/or materials already mentioned in respect of the base component in any of the preceding embodiments.

According to a specific embodiment, the base component is a composite comprising a mesh or an open meshed fabric, in particular made of a polyolefin such as polypropylene, and a non-woven fabric, a felt or a membrane, preferably a membrane, including or predominantly including a protein such as collagen, gelatine, albumin and/or salts thereof. The collagen is preferably selected from the group consisting of collagen type I, collagen type III, salts thereof, stereoisomers thereof, modifications thereof, and combinations thereof. Additionally or alternatively to a protein, the employment of a polysaccharide such as carboxymethylcellulose and/or derivatives thereof may be also conceivable within the scope of this embodiment.

In a further advantageous embodiment, the base component comprises a pocket or is formed as a pocket. In other words, the base component may comprise or be formed as a hollow body defining a lumen which is contained by one layer or, if need be, several layers of the base component. In that regard, the layer or layers of the base component act as wall of the pocket delimiting its lumen. Conveniently, the pocket has an opening which is located on the side of the base component which is adapted to be located towards the inner surface of a cavity wall upon implantation. The pocket advantageously facilitates a flat attachment or alignment of the base component, and thus of the surgical implant according to the present invention, beneath a hernia orifice. The flat attachment and alignment, respectively, may be achieved by a finger of the surgeon or by a suitable instrument.

According to a further convenient embodiment, the pocket as described in the preceding embodiment includes a balloon, for example made of silicon, which may be filled with air, thereby facilitating better placement of the base component, and thus of the surgical implant. After correct placement, the balloon may be removed again.

In a further preferred embodiment, the base component, preferably being formed as mesh or open meshed fabric, may have a coating, for example in the form of a layer, impregnation, film, foil or tape.

The coating may partially or completely cover the base component.

Further, the coating may be present only on one side, in particular only on one two-dimensional side, or on two opposing sides, in particular on two opposing two-dimensional sides, of the base component.

Further, the coating may be non-absorbable, partially absorbable or absorbable.

Furthermore, the coating may have a thickness from 0.01 to 0.75 mm, in particular from 0.05 to 0.4 mm, preferably from 0.1 to 0.25 mm.

Principally, the coating may include or be made of a material selected from the group consisting of polyvinylidene difluoride, polytetrafluorethylene, polytetrafluorpropylene, polyhexafluorpropylene, perfluorinated polyvinyl ethers, poly perfluoroalkoxy perfluorovinyl ethers, polyvinyl pyrrolidone, polyethylene glycol, polypropylene glycol, polypropylene oxide, polytetramethylene oxide, polylactide, polyglycolide, polytrimethylene carbonate, ε-caprolactone, polyhydroxybutyrate, preferably poly-4-hydroxybutyrate, terpolymer made of glycolide, trimethylencarbonate and ε-caprolactone, polyvinyl alcohol, collagen, gelatine, elastin, reticulin, fibronectin, laminin, fibrin, fibrinogen, albumin, starch, amylose, amylopectin, dextran, cellulose, methylcellulose, carboxymethylcellulose, chitosan, hyaluronic acid, dextran sulfate, heparin, heparan sulfate, chondroitin sulfate, dermatan sulfate, copolymers thereof, salts thereof, stereoisomers thereof, and combinations thereof.

Depending on the material used for the coating, the base component may exhibit adhesive or anti-adhesive properties. In other words, it may be possible according to the invention that the base component may have an adhesive or anti-adhesive coating.

Moreover, the coating may have the function to increase stiffness of the base component.

An anti-adhesive coating is especially useful for preventing post-surgical adhesions, i.e. undesired tissue adhesions. Advantageously, the anti-adhesive coating is present on the side of the base component which is adapted to be located towards the interior of a cavity which normally completely contains the protruded organ including a fascia thereof. Preferably, the anti-adhesive coating is present on the side of the base component which is adapted to be located viscerally, i.e. towards viscera and the interior of an abdominal cavity, respectively.

Specifically, the base component may have a non-absorbable and anti-adhesive coating.

In a preferred embodiment, an anti-adhesive coating may be generated onto the base component by subjecting the base component to a treatment with fluorine gas or a gas mixture containing fluorine gas, for example a mixture of fluorine gas and nitrogen gas. Fluorinated implants, particularly in the form of open meshed fabrics, in particular due to a treatment with a mixture containing fluorine gas, are known from EP 2 070 555 A2, whose disclosure insofar is incorporated into the present description by explicit reference.

Alternatively, the base component may be subjected to a hydrophobic surface treatment.

According to an especially preferred embodiment, the base component is a fluorinated mesh or open meshed fabric, in particular being obtainable by treating with fluorine gas or a mixture containing fluorine gas such as a mixture of fluorine gas and nitrogen gas.

In a further embodiment, the anti-adhesive coating may include or be made of a fluorinated, in particular perfluorinated, or fluorine containing polymer which is preferably selected from the group consisting of polyvinylidene difluoride, polytetrafluorethylene, polytetrafluorpropylene, polyhexafluorpropylene, perfluorinated polyvinyl ethers, poly perfluoroalkoxy perfluorovinyl ethers, copolymers thereof, and combinations thereof.

In an alternative embodiment, the anti-adhesive coating may include or be made of poly-4-hydroxybutyrate and/or a terpolymer made of glycolide, trimethylencarbonate and ε-caprolactone. While poly-4-hydroxybutyrate may be preferred if a long-term absorption is desired, a terpolymer a recited in the preceding sentence may be expedient if a short-term absorption is aimed at.

According to an alternate embodiment, the anti-adhesive coating may be a textile coating, preferably in the form of threads which are made of fluorinated or fluorine containing polymers. Further, it may also be within the scope of the present invention that the base component is made of fluorinated or fluorine containing polymers. With respect to useful fluorinated or fluorine-containing polymers as mentioned in this paragraph, reference is made to the preceding embodiment.

An adhesive coating may be advantageous in order to equip the base component with fixating, in particular self-fixating, properties. Preferably, the adhesive coating is absorbable. Absorption of the coating advantageously favours in vivo tissue regeneration and/or remodelling processes, thereby additionally supporting secure fixation of the base component, and thus secure closure of a hernia orifice.

More preferably, the adhesive coating is present on the side of the base component which is adapted to be located towards the inner surface of a cavity wall which normally prevents protrusion of an organ or fascia thereof. Thus, undesired movements of the base component may be prevented, thereby additionally minimizing the risk of recurrent hernias.

Advantageously, the adhesive coating remains attached to the inner surface of the cavity wall until cellular in-growth into the surgical implant has accomplished to such an extent that secure fixation of the base component, and thus of the surgical implant, is no longer dependent on the presence of an adhesive coating.

Especially useful adhesive coatings may be selected from the group consisting of polyvinyl alcohol, collagen, gelatine, salts thereof, and combinations thereof. In that regard, a coating, in particular film, for example made of polyvinyl alcohol, collagen, gelatine and/or salts thereof is especially useful. Besides, reference is made to the materials which have been already enumerated in general for the coating.

In a further embodiment, the base component may have an anti-adhesive coating and an adhesive coating which are preferably present on opposite sides of the base component. The anti-adhesive coating is preferably present on the side of the base component which is located towards the interior of a cavity upon implantation, while the adhesive coating, upon implantation of the surgical implant, is preferably present on the side of the base component which is located to the inner surface of a cavity wall which normally prevents protrusion of an organ or fascia thereof.

In a further useful embodiment, the coating, in particular if present as an adhesive coating, may comprise openings, holes, perforations, or the like. Thus, tissue regeneration and/or remodelling processes may be additionally enhanced.

In order to increase stiffness of the base component, the base component may be alternately embedded in a material. Having regard to useful materials, reference is made to the materials mentioned for the coating in any of the preceding embodiments.

According to an especially preferred embodiment, the base component is selected from the group consisting of an open meshed fabric made of polypropylene, an open meshed fabric made of polypropylene comprising a film made of polyvinyl alcohol, an open meshed fabric made of polypropylene comprising a film made of polydioxanone, an open meshed fabric made of polypropylene comprising a film made of poly-4-hydroxybutyrate, an open meshed fabric made of polypropylene comprising a film made of a terpolymer, in particular a triblock terpolymer, made of glycolide, trimethylene carbonate and ε-caprolactone, an open meshed fabric made of polypropylene comprising a film made of a copolymer made of glycolide and lactide and a composite structure comprising an open meshed fabric and a membrane, a non-woven fabric or a felt including or predominantly including collagen, wherein the collagen is preferably selected from the group consisting of collagen type I, collagen type III, salts thereof, stereoisomers thereof, modifications thereof, and combinations thereof.

In order to increase flexibility, the base component may have cuts, slits, holes, perforations, or the like. Alternatively or in combination, the base component may be finished with softeners or plasticisers such as glycerine.

According to a further advantageous embodiment, the base component comprises retaining elements. The retaining elements advantageously retain the base component, and thus the surgical implant of the present invention, in the correct position upon implantation. Thus, secure fixation of the base component to the cavity wall beneath a hernia orifice may be achieved. Insofar, the retaining elements which may be provided according to the present invention may act as "fixating elements".

Principally, useful retaining elements may be selected from the group consisting of tapes, foils, films, layers, threads, thread parts, barbs, and combinations thereof.

Moreover, the retaining elements may act as self-fixating elements, thereby facilitating self-fixating of the base component and surgical implant according to the present invention, respectively.

Preferably, the retaining elements are arranged as projections or protrusions onto the base component.

More preferably, the retaining elements are arranged as barbs or in the manner of barbs.

Moreover, the retaining elements may be arranged only on one side, particularly only on one two-dimensional side, of the base component. Preferably, the retaining elements are arranged on a side of the base component which is adapted to be located towards the inner surface of a cavity wall which normally prevents protrusion of an organ or fascia thereof.

In principle, the retaining elements and the base component may be made of the same material or of different materials. Further, the retaining elements may be non-absorbable, partially absorbable or absorbable. Absorbable retaining elements advantageously elicit in vivo regeneration and/or remodelling processes.

Having regard to suitable materials for the retaining elements, reference is made to the materials mentioned in respect of the base component.

The retaining elements may be formed as cuts into the base component, thereby preferably generating structures which protrude from the base component.

Alternately or in combination, the retaining elements may be present as textile, in particular thread-like, structures. More specifically, the retaining elements may be selected from the group consisting of threads, cut threads, pile, cut piles, cut pile tufts, pile yarns, plush, thread loops, in particular velour loops, and combinations thereof.

According to an advantageous embodiment, the base component can be made of a non-absorbable material such as polypropylene, while the retaining elements can be made of an absorbable polymer, preferably selected from the group consisting of polyglycolide, polylactide, polydioxanone, polyhydroxybutyrate, poly-3-hydroxybutyrate, poly-4-hydroxybutyrate, polytrimethylencarbonate, poly-E-caprolactone, biopolymers, copolymers, in particular terpolymers, thereof, stereoisomers thereof, salts thereof, and combinations thereof.

It may be further within the scope of the present invention that the base component is a self-fixating textile fabric, in particular a self-fixating mesh, preferably having retaining elements, in particular as described according to any of the preceding embodiments.

In a further embodiment, the base component may have a composite structure comprising a self-fixating textile fabric, in particular a self-fixating open meshed fabric. For example, it may be conceivable according to the present invention that the base component is composed of at least two textile fabrics, wherein one of the at least two textile fabrics is present as a self-fixating textile fabric, preferably as a self-fixating open meshed fabric.

The retaining elements as described in any of the preceding embodiments may form integral parts of the base component or may be fixed to the base component, for instance by means of gluing, welding, in particular ultrasound welding, heat fixing or thermal fixing, stitching, knitting, interlooping, intermeshing, stapling, or the like.

The plug component according to the present invention is advantageously responsible for filling or occluding a hernia orifice. Occlusion of the hernia orifice by means of the plug component may be due to physically and/or chemically fixating.

The plug component may have in principle a textile structure or a non-textile structure.

Preferably, the plug component may be formed as a textile fabric, a mesh or open meshed fabric, a knitted fabric, in particular a warp knitted fabric, a hosiery, a non-woven fabric, a felt, a braided fabric, in particular a rounded or circular braid, or combinations thereof.

In an alternative embodiment, the plug component may be a membrane, sponge, foam, mat, layer, in particular a folded layer, coating, film, foil, or combinations thereof.

Further, the plug component may be a lyophilized shaped or formed body, particularly a lyophilized mat. The plug component may be, by way of example, shaped or formed by means of cold drawing, heat drawing, molding, pressing, stamping, punching, and the like.

In order to additionally foster tissue in-growth and/or tissue remodelling processes, the plug component may be equipped with appropriate agents which will be more detailed in the following.

According to an advantageous embodiment, the plug component comprises or is made of an expandable or deployable material, preferably of a material which is capable of swelling upon contact with body liquids such as blood, lymph, or the like, and/or physiological media such as aqueous solutions, electrolyte solutions, buffer solutions, aqueous dispersions, aqueous suspensions, or the like. Advantageously, such a material may improves adherence to tissue. Useful materials are proteins and/or polysaccharides, in particular as described in the following.

Alternately, the surgical implant, in particular the base component, may have struts, in particular absorbable struts, which self-dependently effect expansion or deployment of the plug component.

The plug component may be in principle non-absorbable, partially absorbable or absorbable. In other words, the plug component may comprise or be made of a non-absorbable, partially absorbable or an absorbable material, in particular polymer.

Preferably, the plug component is absorbable. An absorbable plug component advantageously fosters tissue in-growth and/or tissue remodelling processes within a hernia orifice, thereby supporting natural occlusion of a hernia orifice. This may be additionally supported by finishing the plug component with useful additives, in particular as detailed in the following. Thus, the risk of recurrent hernias can be considerably minimized. With respect to suitable absorbable materials, it is referred to the following description.

In an especially preferred embodiment, the base component is non-absorbable or made of a non-absorbable material, while the plug component is absorbable or made of an absorbable material. With regard to suitable materials for the base component and the plug component, it is referred to the previous and to the following description.

Specifically, the plug component may comprise or be made of a polymer which is selected from the group consisting of polyolefins, polyesters, polyamides, polyurethanes, in particular thermoplastic polyurethanes, polyhydroxyalkanoates, proteins, in particular extracellular proteins, preferably fibrous proteins, globular proteins, polysaccharides, in particular alkylcelluloses, hydroxyalkylcelluloses, carboxyalkylcelluloses, mucopolysaccharides, copolymers, in particular terpolymers, thereof, salts thereof, stereoisomers thereof, and combinations thereof.

More specifically, the plug component may comprise or be made of a polymer which is selected from the group consisting of polyethylene, low-density polyethylene, high-density polyethylene, high-molecular-weight polyethylene, ultra-high-molecular-weight polyethylene, polypropylene, polyethylene terephthalate, polypropylene terephthalate, polybutylene terephthalate, polyamide 6, polyamide 6-6, polyamide 6-12, polyamide 12, polytetrafluorethylene, polyvinylidene dichloride, polyvinylidene difluoride, polytetrafluorpropylene, polyhexafluorpropylene, polyvinyl alcohol, polyglycolide, polylactide, polydioxanone, polyhydroxybutyrate, poly-3-hydroxybutyrate, poly-4-hydroxybutyrate, polytrimethylenecarbonate, poly-ε-caprolactone, polyethylene glycol, polypropylene glycol, collagen, gelatine, elastin, reticulin, fibronectin, laminin, fibrin, fibrinogen, albumin, starch, amylose, amylopectin, dextran, cellulose, methylcellulose, carboxymethylcellulose, chitosan, hyaluronic acid, dextran sulfate, heparin, heparan sulfate, chondroitin sulfate, dermatan sulfate, copolymers, in particular terpolymers, thereof, salts thereof, stereoisomers thereof, and combinations thereof.

A useful homopolymer for the plug component is poly-4-hydroxybutyrate, in particular due to its ability of a long-term absorption.

A useful copolymer for the plug component is a copolymer made of glycolide and lactide, in particular having a weight ratio from 9 : 1 to 1 : 9.

When collagen is used for the plug component, the collagen may be selected from the group consisting of collagen type I, type II, type III, type IV, type V, type VI, type XI, salts thereof, modifications thereof, and combinations thereof. The collagen may be present in a native and/or denatured condition. Typically, the collagen is of xenogenic, in particular porcine, bovine and/or equine, preferably bovine, origin.

According to an alternate especially preferred embodiment, the plug component is a non-woven fabric including or made of a copolymer consisting of gycolide and lactide.

Alternatively, it may be especially preferred that the plug component is an open meshed fabric or a felt made of a polyolefin, in particular made of polypropylene.

According to a further preferred embodiment, the plug component is a non-woven fabric, a felt, a membrane or a foil, preferably a membrane, including or predominantly including a protein such as collagen, gelatine, albumin, salts thereof or combinations thereof. The collagen is preferably selected from the group consisting of collagen type I, collagen type III, salts thereof, stereoisomers thereof, modifications thereof, and combinations thereof. Useful modifications may encompass native and/or denatured conditions.

In a further embodiment, the plug component may be a biological membrane, particularly a membrane of xenogenic origin, for example of porcine, bovine and/or equine origin. More specifically, the plug component may be a pericardium membrane.

According to another embodiment, the plug component, in particular being formed as a synthetic membrane or synthetic film such as a collagen membrane or collagen film, may be cross-linked or non-cross-linked.

Furthermore, the plug component may be a composite, i.e. a structure which is composed of at least two different structures and/or at least two different materials. With respect to useful structures and/or materials for the composite, reference is made in its entirety to the structures and/or materials described in respect of the plug component in any of the preceding embodiments.

Particularly, the plug component is a composite comprising a textile structure and a non-textile structure.

Further, the plug component may be a composite comprising a non-absorbable structure, in particular a non-absorbable textile structure, and an absorbable structure, in particular an absorbable non-textile structure. A non-absorbable textile structure may be beneficially in respect of fixating the plug component to tissue surrounding the hernia orifice.

According to a specific embodiment, the plug component is a composite comprising a mesh or an open meshed fabric, in particular a non-absorbable open-meshed fabric, preferably made of a polyolefin such as polypropylene, and a non-woven fabric, a felt or a membrane, in particular an absorbable non-woven fabric, an absorbable felt or an absorbable membrane, including or predominantly including a protein such as collagen, gelatine, albumin and/or salts thereof. The collagen is preferably selected from the group consisting of collagen type I, collagen type III, salts thereof, stereoisomers thereof, modifications thereof, and combinations thereof. Additionally or alternatively to a protein, the employment of a polysaccharide such as carboxymethylcellulose and/or derivatives thereof may be also conceivable within the scope of this embodiment.

Alternately, the plug component may be a composite comprising a felt made of a material selected from the group consisting of polyvinyl alcohol, collagen, gelatine, salts thereof, and combinations thereof, and a mesh or an open meshed fabric made of a polyolefin, in particular polypropylene.

In a further alternative embodiment, the plug component is a composite comprising to layers of meshes, in particular a layer of a non-absorbable mesh such as a polyolefin mesh, preferably polypropylene mesh, and a layer of an absorbable mesh such as a mesh made of absorbable polyhydroxyester, preferably a mesh made of polyglycolide. For instance, the pug component may comprise a layer of Optilene^{®} Mesh Elastic and a layer of a Safil^{®} mesh (mesh made of Safil^{®} threads).

According to a further alternative embodiment, the plug component is a composite comprising a membrane including or predominantly including collagen, gelatine, albumin and/or salts thereof, and a non-woven fabric made of a copolymer consisting of glycolide and lactide or a non-woven fabric made of polypropylene.

The plug component may further comprise a coating, in particular in the form of a film.

Preferably, the plug component may comprise a coating made of a material selected from the group consisting of polyvinylpyrrolidone, polypropylene glycol, polypropylene oxide, polytetramethylenoxide, polyvinyl alcohol, proteins such as collagen, gelatine, elastin, reticulin, fibronectin, laminin, fibrin, fibrinogen, albumin, polysaccharides such as starch, amylose, amylopectin, dextran, cellulose, methylcellulose, carboxymethylcellulose, chitosan, hyaluronic acid, dextran sulfate, heparin, heparan sulfate, chondroitin sulfate, dermatan sulfate, copolymers thereof, stereoisomers thereof, salts thereof, and combinations thereof.

More preferably, the plug component may comprise a coating made of a material selected from the group consisting of polyvinyl alcohol, polydioxanone, polyhydroxybutyrate, preferably poly-4-hydroxybutyrate, a terpolymer, in particular a triblock terpolymer, made of glycolide, trimethylene carbonate and s-caprolactone, and a copolymer made of glycolide and lactide.

In a more specific embodiment, the plug component, in particular being formed as an open meshed fabric, preferably made of a polyolefin, more preferably made of polypropylene, may comprise a film made of polyvinyl alcohol.

In a preferred embodiment, the plug component may have a pre-shaped or three-dimensional structure. A pre-shaped or three-dimensional plug component facilitates an easier and more convenient handling of the surgical implant according to the invention.

Specifically, the plug component may be shaped as a cone, in particular as a truncated cone. However, also alternative shapes for the plug component may be conceivable according to the present invention. For example, the plug component may be shaped as a cup, funnel, tube, cylinder, or the like. Principally, also combinations of different shapes, in particular as mentioned in this paragraph, may be within the scope of the invention.

Further, the plug component may have a water lily-like cross-section.

According to a further embodiment, the plug component may be kept in a pre-shaped or three-dimensional state, in particular compacted state, for example by means of bonding, gluing, welding, particularly ultrasound welding, heating, thermofixing, clipping, stapling, stitching, knitting, interlooping, intermeshing, or the like.

More specifically, the plug component may be kept in a pre-shaped or three-dimensional state by means of a fixation means, in particular an absorbable fixation means. In general, the fixation means may be selected from the group consisting of a thread, a wire, an adhesive or a glue, in particular tissue adhesive, and combinations thereof.

Preferably, the plug component is kept in a pre-shaped, in particular compacted, state by means of a thread. The thread may be non-absorbable, partially absorbable or absorbable. Preferably, the thread is made of an absorbable material, in particular polymer. Advantageously, the thread is a quickly absorbable thread. More specifically, the thread may be absorbable within a period from 0.01 to 96 days, in particular 0.5 to 60 days, preferably 2 to 40 days. For example, the thread can be made of polyvinyl alcohol such as thermoplastic polyvinyl alcohol. The polyvinyl alcohol preferably has a molecular weight ranging between 25 and 400 kilo Dalton, in particular between 30 and 300 kilo Dalton, preferably between 35 and 250 kilo Dalton.

In an alternate embodiment, the plug component has a planar or two-dimensional structure.

Specifically, the plug component may be formed as a layer, in particular in the form of a coating or film.

Further, the plug component may have a multi-layered or multilaminated structure, i.e. a structure being composed of at least two layers or laminates. For example, the plug component may be composed of four layers, wherein three layers are preferably absorbable and the remaining layer is preferably non-absorbable.

In principle, the base component and the plug component may comprise or may be made of the same material or, if need be, of the same mixture of materials. However, it may be preferable within the scope of the present invention, if the base component and the plug component comprise or are made of different materials or, if need be, of a different mixture of materials. Having regard to useful materials, in particular polymers, reference is made in its entirety to the previous description.

According to a further embodiment, the plug component may comprise retaining elements (fixating elements). Advantageously, the retaining elements prevent a dislocation of the plug component, and thus of the surgical implant according to the present invention. To this means, the retaining means may be attached to the plug component. Suitably attaching techniques may be selected from the group consisting of bonding, gluing, welding, particularly ultrasound welding, heating, thermo fixing, clipping, stapling, stitching, knitting, interlooping, intermeshing, and combinations thereof. Alternatively, the retaining elements may form intergral parts of the plug component.

According to a more specific embodiment, the retaining elements may be selected from the group consisting of tapes, films, foils, layers, threads, thread parts, barbs or barb-like structures, and combinations thereof.

With respect to further features and advantages of the retaining elements for the plug component, it is explicitly referred to the embodiments in respect of the retaining elements for the base component.

According to a further useful embodiment, the implant, in particular the base component and/or plug component, additionally comprises a gripping element (grip element). The gripping element advantageously allows for a more convenient placement of the surgical implant, in particular the base component and/or plug component. For example, the gripping element, being for example arranged in the centre of the plug component, may facilitate a correct placement of the plug component within a hernia orifice.

The gripping element may be especially advantageous where the plug component is present in the form of a flat or two-dimensional plug component. In this case, the gripping element may beneficially support the expansion or deployment of the plug component. In other words, the gripping element may be useful for getting the plug component in the right shape or for building up the plug component.

Furthermore, the gripping element may advantageously act as a fixation means, thereby facilitating fixation of the plug component and the implant, respectively to tissue which, by way of example, surrounds a hernia orifice.

The gripping element may form an integral part of the implant, in particular base component and/or plug component, or may be attached to the implant, in particular base component and/or plug component. For example, the gripping element may be attached to the implant, in particular base component and/or plug component, by means of gluing, welding, in particular ultrasound welding, heat fixing or thermal fixing, stitching, knitting, interlooping, intermeshing, stapling, or the like.

Further, the gripping element may have a textile structure or a non-textile structure.

Accordingly, the gripping element may be a textile structure which is selected from the group consisting of a textile fabric, a textile loop, a mesh or open meshed fabric, a knitted fabric, in particular a warp knitted fabric, a hosiery, a non-woven fabric, a felt, and combinations thereof.

Alternatively, the gripping element may be a non-textile structure which is selected from the group consisting of a membrane, a sponge, a foam, a mat, a layer, a coating, a film, a foil, and combinations thereof.

Further, the gripping element may be a lyophilized shaped or formed body, in particular as mentioned in the preceding paragraph.

According to a further useful embodiment, the gripping element may be a composite, i.e. a structure being composed of at least two different structures and/or at least two different materials. With respect to useful structures and/or materials, reference is made in its entirety to the following description.

A useful composite structure for the gripping element may comprise a textile structure and a non-textile structure. Having regard to useful textile and non-textile structures, reference is made in its entirety to the preceding embodiments.

The gripping element may be principally non-absorbable, partially absorbable or absorbable.

Specifically, the gripping element may comprise or be made of a polymer which is selected from the group consisting of polyolefins, polyesters, polyamides, polyurethanes, in particular thermoplastic polyurethanes, polyhydroxyalkanoates, proteins, in particular extracellular proteins, preferably fibrous proteins, globular proteins, polysaccharides, in particular alkylcelluloses, hydroxyalkylcelluloses, carboxyalkylcelluloses, mucopolysaccharides, copolymers, in particular terpolymers, thereof, stereoisomers thereof, salts thereof, and combinations thereof.

More specifically, the gripping element may comprise or be made of an polymer which is selected from the group consisting of polyethylene, low-density polyethylene, high-density polyethylene, high-molecular-weight polyethylene, ultra-high-molecular-weight polyethylene, polypropylene, polyethylene terephthalate, polypropylene terephthalate, polybutylene terephthalate, polyamide 6, polyamide 6-6, polyamide 6-12, polyamide 12, polytetrafluorethylene, polyvinylidene dichloride, polyvinylidene difluoride, polytetrafluorpropylene, polyhexafluorpropylene, polyvinyl alcohol, polyglycolide, polylactide, polydioxanone, polyhydroxybutyrate, poly-3-hydroxybutyrate, poly-4-hydroxybutyrate, polytrimethylene-carbonate, poly-s-caprolactone, polyethylene glycol, polypropylene glycol, collagen, gelatine, elastin, reticulin, fibronectin, laminin, fibrin, fibrinogen, albumin, starch, amylose, amylopectin, dextran, cellulose, methylcellulose, carboxymethylcellulose, chitosan, hyaluronic acid, dextran sulfate, heparin, heparan sulfate, chondroitin sulfate, dermatan sulfate, copolymers, in particular terpolymers, thereof, stereoisomers thereof, salts thereof, and combinations thereof.

A useful copolymer for the gripping element is a copolymer made of glycolide and lactide, in particular having a weight ratio from 9 : 1 to 1 : 9.

A useful terpolymer for the gripping element is a terpolymer, in particular a triblock terpolymer, made of glycolide, trimethylene carbonate and s-caprolactone. As already mentioned, such a terpolymer is commercially available under the name Monosyn^{®}. Such a terpolymer has the advantage that it is typically absorbed within 60 to 90 days.

When collagen is used for the gripping element, the collagen may be selected from the group consisting of collagen type I, type II, type III, type IV, type V, type VI, type XI, salts thereof, modifications thereof, and combinations thereof. Typically, the collagen is of xenogenic, in particular porcine, bovine and/or equine, preferably bovine, origin.

According to an especially preferred embodiment, the gripping element is a mesh or open meshed fabric, in particular a non-absorbable mesh or open meshed fabric, made of a polyolefin such as polypropylene.

According to an alternative especially preferred embodiment, the gripping element is a non-woven fabric, felt, membrane or foil including or predominantly including a protein, in particular collagen, gelatine, albumin and/or salts thereof. The collagen is preferably selected from the group consisting of collagen type I, collagen type III, salts thereof, stereoisomers thereof, modifications thereof, and combinations thereof. In a further embodiment, the gripping element comprises a mesh, in particular a non-absorbable mesh such as a polypropylene mesh, and a coating, in particular a film. The coating is preferably made of a material, typically polymer, which may be selected from the group consisting of polyvinylpyrrolidone, polyethylene glycol, polypropylene glycol, polypropylene oxide, polytetramethylenoxide, polyvinyl alcohol, proteins such as collagen, gelatine, elastin, reticulin, fibronectin, laminin, fibrin, fibrinogen, albumin, polysaccharides such as starch, amylose, amylopectin, dextran, cellulose, methylcellulose, carboxymethylcellulose, chitosan, hyaluronic acid, dextran sulfate, heparin, heparan sulfate, chondroitin sulfate, dermatan sulfate, copolymers thereof, stereoisomers thereof, salts thereof, and combinations thereof.

Alternatively, it may also be conceivable according to the invention that the gripping element is embedded in a material, in particular in at least one material as enumerated in the preceding embodiment.

More preferably, the gripping element comprises an open meshed fabric, in particular a non-absorbable open meshed fabric, preferably made of a polyolefin such as polypropylene, and a non-woven fabric, a felt or a membrane preferably including or predominantly including a protein such as collagen, gelatine, albumin and/or salts thereof. The collagen is preferably selected from the group consisting of collagen type I, collagen type III, salts thereof, stereoisomers thereof, modifications thereof, and combinations thereof. Additionally or alternatively to a protein, the employment of a polysaccharide such as carboxymethylcellulose and/or derivatives thereof may be also conceivable within the scope of this embodiment.

According to a further embodiment, the surgical implant, in particular the base component, plug component and/or an optionally provided gripping element, may be finished with additives such as active agents, in particular biological agents, pharmaceutical agents, medicinal agents, cells, in particular stem cells like mesenchymal stem cells, radiopaque additives, marking additives, dyes, softening agents, or combinations thereof.

Specifically, the surgical implant, in particular the base component, plug component and/or an optionally provided gripping element, may be soaked with additives such as mentioned in the preceding embodiment.

Preferably, the surgical implant, in particular the base component, plug component and/or an optionally provided gripping element, is finished with additives which are preferably promoting the healing and occlusion, respectively of a hernia orifice. Having regard to useful agents, reference is made to the following embodiments.

According to a more specific embodiment, the surgical implant, in particular the base component, plug component and/or an optionally provided gripping element, may comprise active agents which are selected from the group consisting of antimicrobial agents, in particular antibiotics, disinfectants, anti-scarring agents such as captopril, antiinflammatory agents, growth factors, cellular adhesion agents, cellular recruiting agents, cytokines, peptides, proteins, such as collagen, lipids, polysaccharides such as hyaluronic acid, platelet-rich plasma (PRP), oligonucleotides, polynucleotides, DNA, RNA, cellular receptors, stereoisomers thereof, salts thereof, and combinations thereof.

Useful antimicrobial agents may be selected from the group consisting of copper, silver, gold, triclosan, biguanide, chlorhexidine, polyhexame-thylenbiguanide, salts thereof, and combinations thereof.

In order to facilitate a fast healing of a hernia orifice, platelet-rich plasma (PRP) is especially preferred. The term "platelet-rich plasma" (PRP) relates to blood plasma which is enriched with platelets. The platelets include a variety of cytokines which stimulate the healing of soft tissue. With respect to cytokines which may be included in platelet-rich plasma (PRP), it is referred to the following embodiment.

According to an especially preferred embodiment, the plug component is finished with platelet-rich plasma, in particular autologous platelet-rich plasma.

Suitable growth factors may be selected from the group consisting of fibroblastic growth factor (FGF), transforming growth factor (TGF), plate-late derived growth factor (PDGF), epidermal growth factor (EGF), granulocyte-macrophage colony stimulation factor (GMCSF), vascular endothelial growth factor (VEGF), insuline-like growth factor (IGF), hepatocyte growth factor (HGF), interleucine-1B (IL-1B), interleucine-8 (IL-8), nerve growth factor (NGF), and combinations thereof.

Further, the additives may be present as micro- and/or nanoparticles.

In order to soften the surgical implant, in particular the base component, plug component and/or an optional gripping element, it may be within the scope of the present invention to pre-wet the implant, in particular the base component, plug component and/or an optional gripping element. Such a pre-wetting may be achieved by equipping or finishing the implant, in particular base component, plug component and/or an optional gripping element, with a liquid such as a solution, suspension or liquid dispersion. Typically, an aqueous liquid such as aqueous solution, aqueous suspension or aqueous dispersion is applied. Further, the liquid may include additives, in particular as described in the preceding embodiments, wherein a PRP including liquid is especially preferred.

In a further embodiment, the surgical implant may act as a drug delivery system. Regarding useful drugs, it is referred to the active agents enumerated in the preceding embodiments.

Principally, the base component, plug component and/or an optionally provided gripping element may have a circular or a non-circular shape. Having regard to a non-circular shape, an oval shape, an ellipsoid shape or a polygonal shape may be conceivable according to the present invention. Useful polygonal shapes may be selected from the group consisting of triangular shape, square shape, rectangular shape, trapezoid shape, rhomboid shape, diamond-like shape, pentagonal shape, hexagonal shape and star-like shape.

A further aspect of the present invention relates to a surgical combination, in particular a surgical kit, comprising a surgical implant and a delivery instrument. The delivery instrument allows for delivering the implant into the body of a human or animal subject in such a way that successful treatment of a hernia defect is possible. The delivery instrument may be, by way of example, an overtube or catheter.

With respect to further advantages and features of the surgical implant, reference is made in its entirety to the previous description.

Further advantages and features of the invention will become clear from the following description of the drawings and from the example in conjunction with the dependent claims. The individual features can be realized either singly or separately in combination in one embodiment of the invention. The drawings and the examples merely serve for illustration and better understanding of the invention and are not to be understood as in any way limiting the invention.

The figures schematically show:
- Fig. 1:: a hernia defect,
- Fig. 2: the position of an embodiment of the surgical implant used for hernia management according to the invention upon placement into the body of a subject,
- Fig. 3a:: an exploded side view of an embodiment of the surgical implant according to the invention,
- Fig. 3b:: a top view of the surgical implant shown in Fig. 3a,
- Fig. 4a:: an exploded side view of a further embodiment of the surgical implant according to the invention,
- Fig. 4b:: a top view of the surgical implant as shown in Fig. 4a,
- Fig. 5:: an exploded side view of a further embodiment of the surgical implant according to the invention,
- Fig. 6:: a side view of a further embodiment of the surgical implant according to the invention,
- Fig. 7a:: a side view of a further embodiment of the surgical implant according to the invention,
- Fig. 7b:: a top view of the surgical implant as shown in Fig. 7a,
- Fig. 8a:: a side view of a further embodiment of the surgical implant according to the invention,
- Fig. 8b:: a top view of the surgical implant as shown in Fig. 8a,
- Fig. 9a:: a side view of a further embodiment of the surgical implant according to the invention,
- Fig. 9b:: a top view of the surgical implant as shown in Fig. 9a,
- Fig. 10a:: a side view of a further embodiment of the surgical implant according to the invention,
- Fig. 10b:: a top view of the surgical implant as shown in Fig. 10a,
- Fig. 11a:: a side view of a further embodiment of the surgical implant according to the invention,
- Fig. 11b:: a top view of the surgical implant as shown in Fig. 11a,
- Fig. 12:: a side view of a further embodiment of the surgical implant according to the invention,
- Fig. 13a-c:: side view of further embodiments of the surgical implant according to the invention,
- Fig. 14:: a side view of a further embodiment of the surgical implant according to the invention.

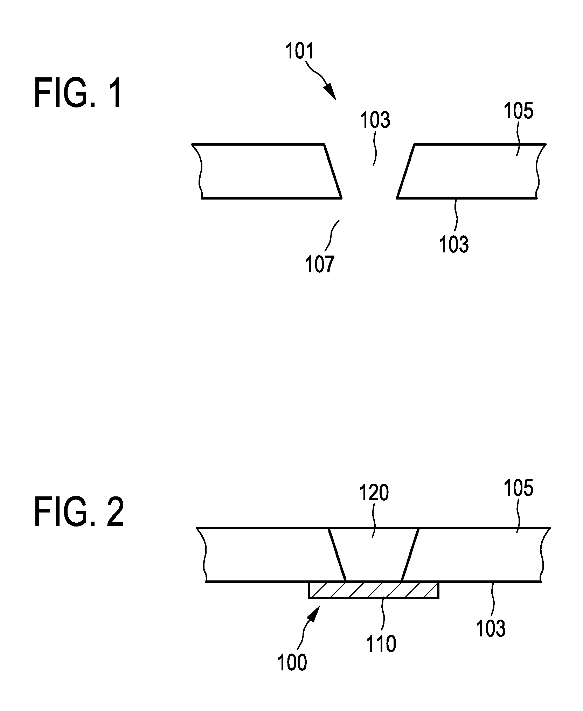

Figure 1 schematically shows a hernia defect 101. The hernia defect 101 is typically featured by a hernia orifice 103 which is due to protrusion of an organ or facia thereof (not shown) through the wall 105 of a cavity 107. The inner surface 103 of the wall 105 is directed towards the interior of the cavity 107.

Figure 2 schematically displays a surgical implant 100 comprising a base component 110 being typically formed as two-dimensional shaped body such as a mesh and comprising a plug component 120, wherein the plug component 120 preferably has a truncated cone shape. The implant 100 is typically delivered into the body of a subject in such a fashion that the base component 110 is located towards the inner surface 103 of the cavity wall 105, while the plug component 120 is located within the hernia orifice 103.

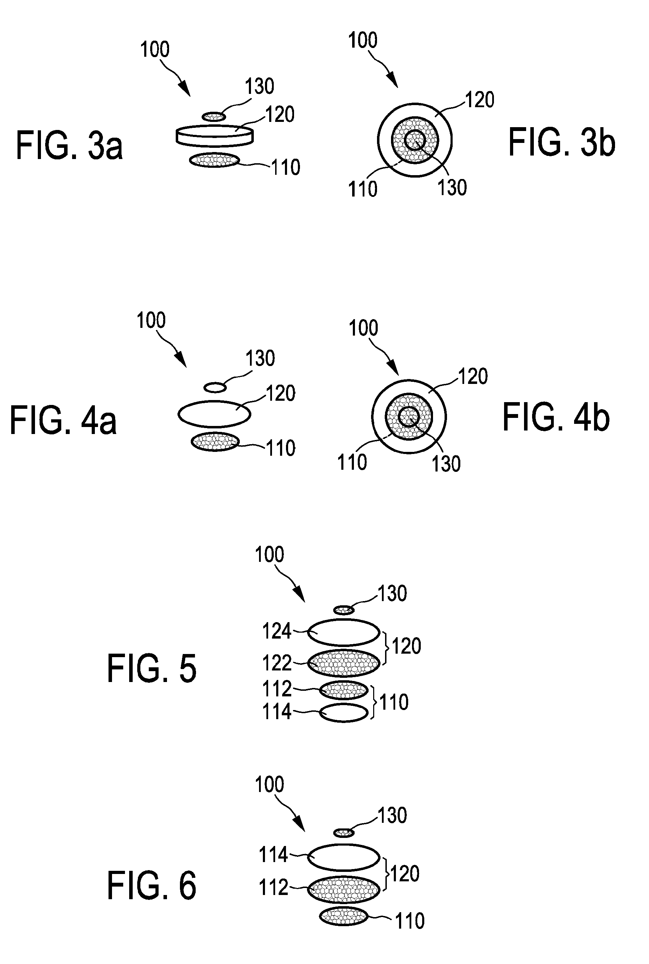

Figures 3a and 3b schematically show a surgical implant 100 comprising a non-absorbable base component 110, an absorbable plug component 120 and a non-absorbable gripping element 130. The base component 110 and the gripping element 130 are each preferably a polypropylene mesh, while the plug component 120 preferably is a collagen nonwoven. The gripping element 130 may be basically attached to the centre of the plug component 120 and/or the base component 110.

The gripping element 130 may not only be useful for building up or expanding the plug component 120 but also for fixating the surgical implant 100 to tissue being in the vicinity of a hernia orifice.

Further, the plug component 120 may be soaked with additives such as active agents like platelet-rich plasma and/or cells.

Figure 4a and 4b schematically show a surgical implant 100 comprising a non-absorbable base component 110, an absorbable plug component 120 and an absorbable gripping element 130. The base component 110 is preferably a mesh made of polypropylene. The plug component 120 and the gripping element 130 are each preferably a membrane including or predominantly including collagen. The gripping element 130 may be basically attached to the centre of the plug component 120 and/or the base component 110.

Figure 5 schematically shows a surgical implant 100 comprising a partially absorbable base component 110, a partially absorbable plug component 120, and a non-absorbable gripping element 130. The base component 110 and the plug component 120 each have a composite structure which is preferably composed of a polypropylene mesh 112; 122 and a collagen membrane 114; 124. Within the implant structure the polypropylene mesh 112 of the base component 110 and the polypropylene mesh 122 of the plug component 120 are adjacent to each other.

The gripping element 130 which is preferably non-absorbable may be attached to the centre of the collagen membrane 124 and/or to the centre of the mesh 122 of the plug component 120 and/or fixed to the base component 110.

Figure 6 schematically shows a surgical implant 100 comprising a non-absorbable base component 110, a partially absorbable plug component 120 and a non-absorbable gripping element 130. The base component 110 is preferably a mesh made of polypropylene. The plug component 120 has a composite structure which is preferably composed of a membrane 124 which may include or predominantly include collagen and of a polypropylene mesh 122. The gripping element 130 is preferably a polypropylene mesh. Within the implant structure, the base component 110 is preferably attached to the polypropylene mesh 122 of the plug component 120, while the gripping element 130 which is preferably non-absorbable may be basically attached to the centre of the membrane 124 and/or to the centre of the mesh 122 of the plug component 120.

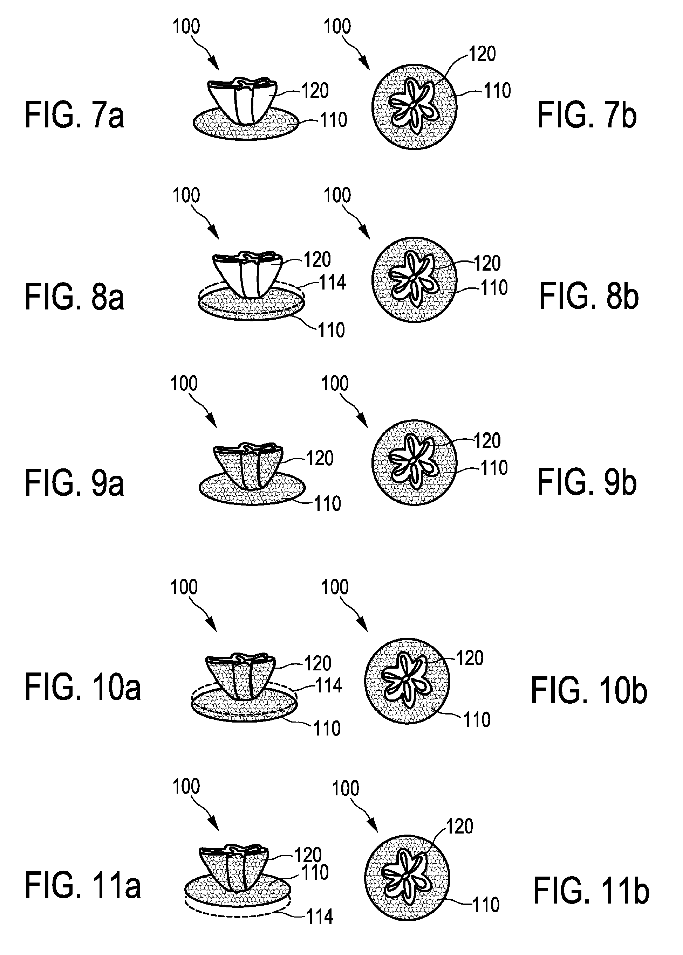

Figures 7a and 7b schematically show a surgical implant 100 comprising a non-absorbable base component 110, preferably in the form of a polypropylene mesh, and a pre-shaped plug component 120 which may have a lily-like cross-section (Figure 5b) and may be a non-woven fabric including or predominantly including collagen and/or a copolymer made of glycolide and lactide. Such a plug component is especially useful in respect of finishing the plug component with additives.

Figures 8a and 8b schematically show a surgical implant 100 comprising a partially absorbable base component 110 and an absorbable, pre-shaped plug component 120. The base component 110 may be a polypropylene mesh which is layered by a film 114 (depicted as circle in slashed line) which is preferably made of a material which is selected from the group consisting of polyvinyl alcohol, polydioxanone, a terpolymer made of glycolide, trimethylene carbonate and ε-caprolacton, and a copolymer made of glycolide and lactide.

The plug component 120 may be a pre-shaped film which is preferably made of a material which is selected from the group consisting of polyvinyl alcohol, polydioxanone, a terpolymer made of glycolide, trimethylene carbonate and ε-caprolacton, and a copolymer made of glycolide and lactide. In order to increase flexibility and to promote cellular in-growth, the plug component 120 may have holes, openings, perforations, and the like.

The Figures 9a and 9b schematically show a surgical implant 100 comprising a non-absorbable base component 110 and a pre-shaped and partially absorbable plug component 120. The base component 110 is preferably a polypropylene mesh, while the plug component 120 may have a composite structure which is preferably composed of a membrane including or predominantly including collagen and of a polypropylene mesh.

Figures 10a and 10b schematically show a surgical implant 100 comprising a partially absorbable base component 110 and a partially absorbable plug component 120. The base component 110 is preferably an open-meshed fabric made of polypropylene, wherein the fabric is layered by an absorbable film 114 (depicted as circle in slashed line) which, for example, is made of polyvinyl alcohol and/or carboxymethylcellulose.

The plug component 120 may be a composite which may be composed of an open meshed fabric made of polypropylene which is layered by a film made of polyvinyl alcohol. As an alternative, the plug component 120 may be a composite composed of an open meshed fabric which may be made of polypropylene and of a felt which may be made of polyvinyl alcohol and/or carboxymethylcellulose.

Figures 11a und 11b schematically show a surgical implant 100 comprising a partially absorbable base component 110 and a pre-shaped and partially absorbable plug component 120. The base component 110 and the plug component 120 each are preferably formed as a polypropylene mesh which is layered by a film made of polyvinyl alcohol (not shown). Additionally or alternatively to the polyvinyl alcohol film, the base component may have a coating 114 of a terpolymer made of glycolide, trimethylene carbonate and ε-caprolactone. The coating 114 is expediently directed towards the interior of a body cavity upon implantation.

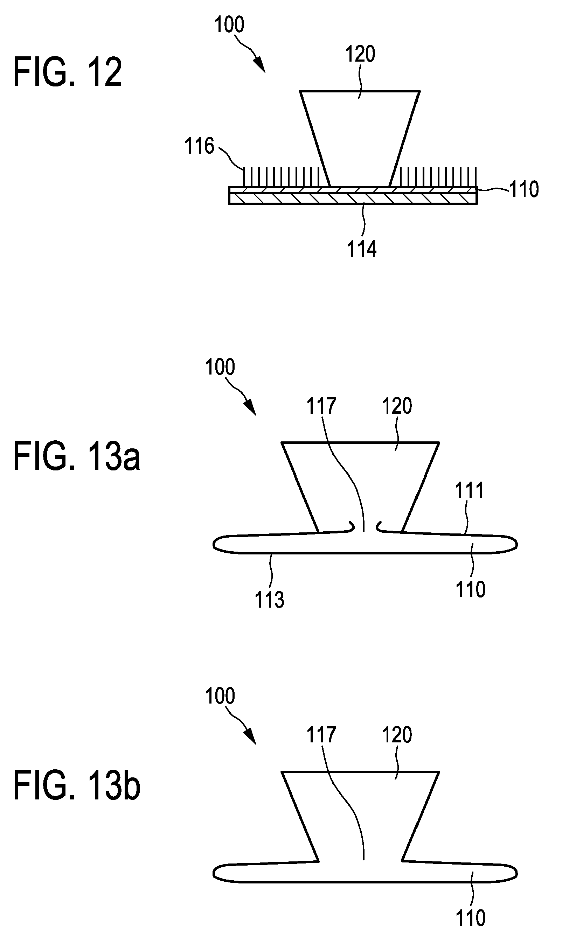

Fig. 12 schematically shows a side view of a surgical implant 100 comprising a base component 110 and a pre-shaped plug component 120.

The base component 110 comprises retaining elements 116, preferably formed as barbs. Conveniently, the retaining elements 116 are disposed on the side of the base component 110 which is directed towards the inner surface of a cavity wall upon implantation. The retaining elements 116 may be advantageously pressed into an inner cavity wall, thereby facilitating fixating of the base component 110 and the surgical implant 100, respectively.

Instead of the retaining elements 116, the base component 110 may comprise an adhesive membrane (not shown).

In order to avoid tissue, particularly visceral, adhesions, the surgical implant 100 may additionally comprise an anti-adhesive coating 114.

Fig. 13a schematically displays a side view of a surgical implant 100 comprising a base component 110 being formed as a pocket and a pre-shaped plug component 120. The pocket 110 has an opening 117. Typically, the opening 117 is formed on the base component 110 adjacent to the plug component 120.

Further, the base component 110 may be composed of an absorbable layer 111 and a non-absorbable layer 113. Advantageously, the absorbable layer 111 is directed towards the inner surface of a cavity wall upon implantation, while the non-absorbable layer 113 is expediently directed to the interior of a cavity.

As an alternative, the opening 117 may be bordered by the plug component 120 (Fig. 13b).

Due to the pocket structure of the base component 110, a flat attachment and alignment, respectively, of the base component 110 beneath a hernia orifice may be achieved. In that regard, a finger of the surgeon or a suitable instrument may be used to flatten and attach the base component 110 beneath the hernia orifice.

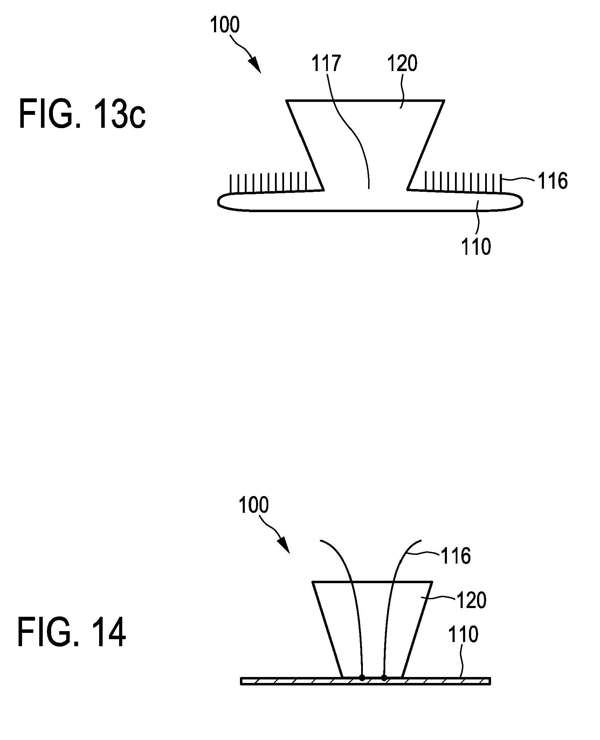

In order to improve fixation of the base component 110 to the inner surface of a cavity wall, the base component 110 may have retaining elements 116 as shown in Fig. 13c. In addition or as an alternative to retaining elements, the side of the base component 110 which is located towards the inner surface of a cavity wall upon implantation may be coated with an adhesive material. Furthermore, the opposing side of the base component 110 may be coated with an anti-adhesive material so as to avoid adhesions with tissue like viscera.

Fig. 14 schematically shows a side view of a surgical implant 100 comprising a base component 110, a pre-shaped plug component 120 and retaining elements 116 which may be formed as threads. The retaining elements 116 may be attached to the base component 110.

The retaining elements 116 are responsible for holding the base component 110, and thus the surgical implant 100, in the correct position after implantation. In other words, the retaining elements 116 advantageously prevent an undesired dislocation of the base component 110 and surgical implant 100, respectively. In order to reduce input of foreign material into the body of a subject, the retaining elements 116 may be made of an absorbable material.

With respect to further advantages and features of the implant, in particular in respect of its components and elements, respectively, reference is made in its entirety to the preceding description.

### Example: Manufacture of an anti-adhesive surgical implant comprising a non-absorbable base component and a partially absorbable plug component

A textile mesh which is commercially available under the name Optilene^{®} Mesh Elastic was laser cut into rondelles having a diameter of 65 mm.

Further, meshes made of Safil^{®} threads were stamped out, thereby creating rondelles having a diameter of 75 mm, in the following denoted as Safil^{®} rondelles.

The Safil^{®} rondelles were placed beneath the Optilene^{®} Mesh Elastic rondelles and fixed to each other by applying a temperature range from 190 to 200°C and a pressure of 1,5 bar during 2 seconds (thermal fixating). As regards the obtained base component, the Safil^{®} rondelles overlapped the Optilene^{®} Mesh Elastic rondelles and acted as an anti-adhesive absorbable layer.

Furthermore, a composite plug component being composed of Optilene^{®} Mesh LP and a mesh made of Safil^{®} threads, in the following denoted as Safil^{®} mesh, was used for the production of the surgical implant. To form a composite, the Optilene^{®} Mesh LP and Safil^{®} mesh were pointwise fixed together by means of ultrasound, wherein the mutual distance of the fixating points was 8 mm. Then, rondelles having a diameter of 50 mm were stamped out. Afterwards, the composite rondelles were shaped to a cone, wherein the absorbable Safil^{®} mesh was directed to the interior of the cone. The cone shape was further fixed by a Safil^{®} quick thread having a size of USP 5-0.

Thereafter, the head of the cone-shaped plug component was sutured to the base component using a Premilene^{®} thread (USP 4-0), thereby yielding a three-dimensional surgical implant being especially useful for repairing hernia defects.

## Claims

1. Surgical implant (100), in particular for use as a hernia repair implant, comprising a base component (110) and a plug component (120), **characterized in that** the base component (110) is adapted to be located beneath a hernia orifice and the plug component (120) is adapted to be placed within a hernia orifice.

2. Surgical implant (100) according to claim 1, **characterized in that** the base component (110) and the plug component (120) are attached to each other by means of bonding, gluing, welding, particularly ultrasound welding, heating, thermofixing, clipping, stapling, stitching, knitting, intermeshing, interloping, and combinations thereof.

3. Surgical implant (100) according to claim 1 or 2, **characterized in that** the base component (110) is a textile fabric, preferably selected from the group consisting of an open-meshed fabric, a knitted fabric, in particular warp knitted fabric, a hosiery, a non-woven fabric, a felt, a braided fabric, and combinations thereof.

4. Surgical implant (100) according to any of the preceding claims, **characterized in that** the base component (110) is made of a material, in particular polymer, selected from the group consisting of polyethylene, low-density polyethylene, high-density polyethylene, high-molecular-weight polyethylene, ultra-high-molecular-weight polyethylene, polypropylene, polyethylene terephthalate, polypropylene terephthalate, polybutylene terephthalate, polyamide 6, polyamide 6-6, polyamide 6-12, polyamide 12, polytetrafluorethylene, polyvinylidene dichloride, polyvinylidene difluoride, polytetrafluorpropylene, polyhexafluorpropylene, polyvinyl alcohol, polyglycolide, polylactide, polydioxanone, polyhydroxybutyrate, poly-3-hydroxybutyrate, poly-4-hydroxybutyrate, polytrimethylenecarbonate, poly-s-caprolactone, polyethylene glycol, proteins such as collagen, gelatine, elastin, reticulin, fibronectin, laminin, fibrin, fibrinogen, albumin, polysaccharides such as starch, amylose, amylopectin, dextran, cellulose, methylcellulose, carboxymethylcellulose, chitosan, hyaluronic acid, dextran sulfate, heparin, heparan sulfate, chondroitin sulfate, dermatan sulfate, copolymers thereof, stereoisomers thereof, salts thereof, and combinations thereof.

5. Surgical implant (100) according to any of the preceding claims, **characterized in that** the base component (110) is an open meshed fabric, in particular a non-absorbable open meshed fabric, preferably made of a polyolefin, more preferably made of polypropylene.

6. Surgical implant (100) according to any of the preceding claims, **characterized in that** the base component (110) comprises an open meshed fabric, in particular a non-absorbable open meshed fabric, particularly made of polyolefin such as polypropylene, and a coating, wherein the coating is preferably made of a polymer selected from the group consisting of polyvinylpyrrolidone, polyethylene glycol, polypropylene glycol, polypropylene oxide, polytetramethylene oxide, poly-4-hydroxybutyrate, terpolymer made of glycolide, trimethylene carbonate and ε-caprolactone, polyvinyl alcohol, collagen, gelatine, elastin, reticulin, fibronectin, laminin, fibrin, fibrinogen, albumin, starch, amylase, amylopectin, dextran, cellulose, methylcellulose, carboxymethylcellulose, chitosan, hyaluronic acid, dextran sulphate, heparin, heparan sulfate, chondroitin sulfate, dermatan sulphate, copolymers thereof, stereoisomers thereof, salts thereof, and combinations thereof.

7. Surgical implant (100) according to any of the claims 1 to 5, **characterized in that** the base component (110) comprises an open meshed fabric, in particular a non-absorbable open meshed fabric, preferably made of a polyolefin such as a polypropylene, and a non-woven fabric, felt, membrane or foil, in particular including or predominantly including a protein such as collagen, gelatine, albumin and/or salts thereof.

8. Surgical implant (100) according to any of the preceding claims, **characterized in that** the base component (110) is formed as a pocket.

9. Surgical implant (100) according to any of the preceding claims, **characterized in that** the base component (110) comprises retaining elements (116), in particular barbs or in the manner of barbs, wherein the retaining elements (116) are preferably arranged on a side of the base component which is adapted to be located towards the inner surface of a body cavity wall.

10. Surgical implant (100) according to any of the preceding claims, **characterized in that** the plug component (120) is pre-shaped or three-dimensionally formed, wherein the plug component (120) is preferably shaped as a cone, in particular as a truncated cone.

11. Surgical implant (100) according to any of the preceding claims, **characterized in that** the plug component (120) is kept in a pre-shaped or three-dimensional state by means of a fixations means, in particular an absorbable fixation means such as an absorbable thread.

12. Surgical implant (100) according to any of the preceding claims, **characterized in that** the implant (100) further comprises a gripping element (130), preferably being attached to a planar or two-dimensionally shaped plug component.

13. Surgical implant (100) according to claim 12, **characterized in that** the gripping element (130) is an open meshed fabric, in particular made of a polyolefin such as polypropylene, or a membrane including or predominantly including a protein such as collagen, gelatine, albumin and/or salts thereof.

14. Surgical implant (100) according to any of the preceding claims, **characterized in that** the plug component (120) is selected from the group consisting of an open meshed fabric, a non-woven fabric, a felt, a membrane, a sponge, a foam, a layer, a coating, a film, a foil, and combinations thereof.

15. Surgical implant (100) according to any of the preceding claims, **characterized in that** the plug component (120) comprises or is made of a material, in particular polymer, selected from the group consisting of polyethylene, low-density polyethylene, high-density polyethylene, high-molecular-weight polyethylene, ultra-high-molecular-weight polyethylene, polypropylene, polyethylene terephthalate, polypropylene terephthalate, polybutylene terephthalate, polyamide 6, polyamide 6-6, polyamide 6-12, polyamide 12, polytetrafluorethylene, polyvinylidene dichloride, polyvinylidene difluoride, polytetrafluorpropylene, polyhexafluorpropylene, polyvinyl alcohol, polyglycolide, polylactide, polydioxanone, polyhydroxybutyrate, poly-3-hydroxybutyrate, poly-4-hydroxybutyrate, polytrimethylenecarbonate, poly-s-caprolactone, collagen, gelatine, elastin, reticulin, fibronectin, laminin, fibrin, fibrinogen, albumin, starch, amylose, amylopectin, dextran, cellulose, methylcellulose, carboxymethylcellulose, chitosan, hyaluronic acid, dextran sulfate, heparin, heparan sulfate, chondroitin sulfate, dermatan sulfate, copolymers thereof, stereoisomers thereof, salts thereof, and combinations thereof.

16. Surgical implant (100) according to any of the preceding claims, **characterized in that** the plug component (120) is a non-woven fabric, a felt or a membrane including or predominantly including a protein such as collagen, gelatine, albumine and/or salts thereof.

17. Surgical implant (100) according to any of the preceding claims, **characterized in that** the plug component (120) comprises an open-meshed fabric, in particular a non-absorbable open-meshed fabric, in particular made of polyolefin such as polypropylene, and a coating, wherein the coating is preferably made of a polymer selected from the group consisting of polyvinylpyrrolidone, polypropylene glycol, polypropylene oxide, polytetramethylenoxide, polyvinyl alcohol, proteins such as collagen, gelatine, elastin, reticulin, fibronectin, laminin, fibrin, fibrinogen, albumin, polysaccharides such as starch, amylose, amylopectin, dextran, cellulose, methylcellulose, carboxymethylcellulose, chitosan, hyaluronic acid, dextran sulfate, heparin, heparan sulfate, chondroitin sulfate, dermatan sulfate, copolymers thereof, stereoisomers thereof, salts thereof, and combinations thereof.

18. Surgical implant (100) according to any of the preceding claims, **characterized in that** the plug component (120) is a composite comprising an open meshed fabric, in particular a non-absorbable open meshed fabric, preferably made of a polyolefin such as polypropylene, and a membrane, in particular an absorbable membrane, including or predominantly including a protein such as collagen, gelatine, albumin and/or salts thereof.

19. Surgical combination, in particular surgical kit, comprising a surgical implant (100) according to any of the preceding claims and a delivery instrument such as an overtube or catheter.
